# EUROPEAN PATENT APPLICATION

(11) **EP 3 282 020 A1**
(43) Date of publication of application: **14.02.2018**
(21) Application number: 16183555.8
(22) Date of filing: 10.08.2016
(51) Int. Cl.: C12Q 1/68

(54) **METHOD FOR AMPLIFYING SIGNALS FROM SINGLE MOLECULES AND SYSTEM OR KIT THEREFORE**

(71) Applicant: Technische Universität Braunschweig, 38106 Braunschweig (DE)
(72) Inventor: TINNEFELD, Philip, Prof. Dr., 38106 Braunschweig (DE); VIETZ, Carolin, 38106 Braunschweig (DE); LALKENS, Birka, Dr., 38106 Braunschweig (DE)
(74) Representative: Kröncke, Rolf

(57) **Abstract**

In a first aspect, the present invention relates to a method for amplifying a signal based on a single target molecule containing a target sequence applying the DNA walker technique. In addition, the present invention provides a kit or system for signal enhancement, in particular, for signal enhancement of chromophores like fluorophores.

## Description

In a first aspect, the present invention relates to a method for amplifying a signal based on a single target molecule containing a target sequence applying the DNA walker technique. In addition, the present invention provides a kit or system for signal enhancement, in particular, for signal enhancement of chromophores like fluorophores.

### Prior art

Single molecule techniques have a substantial influence on the developments in the nanobiotechnology field, such a super-resolution microscopy or single molecule DNA sequencing. Also in the field of molecular diagnostics single molecules, like marker molecules, need to be detected specifically. In this connection, difficulties occur on the one hand in detecting specifically a single molecule even at low concentration and on the other hand in detecting the signal but not detecting surrounding molecules accordingly.

Often, DNA molecules are determined as marker molecules being specific for diseases' or as a biomarker in the diagnostic field. For detecting these markers often optical method are used including methods based on chromophores like fluorophores. However, the signal of said chromophores like fluorophores at low concentrations is a main problem in the art since most of the detections systems are not equipped with suitable means for determining the signals at low levels. Thus, amplification and enhancement of the signal is required allowing to obtain a distinct result.

Various methods are described in the art allowing signal enhancement and signal amplification for molecular diagnostics. Most of them are based on enzymatic amplification. The most prominent one is the polymerase chain reaction, PCR, where DNA molecules are amplified by polymerase in various cycles and the amplified molecules are detected thereafter. In addition, signal enhancement in molecular assays are described e.g. in US 2013/0252825 where the signal enhancement is possible by plasmon resonance. However, a problem of this technique is to position the marker molecule exactly in the field of plasmon resonance, thus, allowing electrical filed enhancement of the signal accordingly. Recently, Puchkova, A., et al., Nano Lett. 2015, 15, 8354-8359, described DNA origami nanoantennas with over 5000-fold fluorescence enhancement and single-molecule detection at 25 µm. Therein, enhancement is possible by plasmon resonance.

The methods known in the art have various advantages and disadvantages. For example, most of the techniques involves laborious steps and expensive equipment as well as well-trained staff. In addition, quite often laborious purification step are required before allowing detection of the maker molecules accordingly. Thus, the known methods for single molecule detection need further improvement.

Self-assembled DNA structures, termed DNA-origami-technique, were first described by Rothemund et al., see WO 2005/030978. The main concept consists and providing a single DNA strand, typically of more than 5000 base pairs, termed scaffold, folded with the help of several (typically more than 100) different staple strands. The self-assembly DNA structure is formed by mixing the single DNA strand together with staples strands, thus, forming the predetermined DNA structure. The so called DNA origami technique enables nano scaffolding of the DNA to create arbitrary two and three dimensional shapes at the nanoscale.

In the US 2013/0252825 A1 this technique is used to allow signal enhancement by plasmon resonance accordingly.

In EP 2 940 150 A1 methods for detecting an analyte in a sample utilizing self-assembly of nucleic acids to yield predetermined nanostructures are described. The analyte is present if a nanostructure is formed.

A DNA walker is a class of nucleic acid nanomachines where a nucleic acid sequence, a so called "walker", is able to move along a track formed of nucleic acids. Already various applications of these kind of molecules are described in the art including nanomedicine, diagnostic sensing of biological samples etc. These DNA walker were described e.g. Wickham et al., Nat nanotechnol 2011, 6, 166-169.

It is the aim of the present invention to overcome the limitations by known methods and to improve the detection of molecules with low concentrations including single molecules, like chromophores including fluorophores, thus, a strong signal can be detected improving the detection of distinct signals at low concentrations. In particular, the aim of the present invention is to provide a method where locally the signal is enhanced, thus, a single signal, like a marker, e.g. a label, can be detected locally at low concentration by signal enhancement. That is, the aim of the present invention is to provide methods useful in detecting molecules at low concentration or single molecule studies by signal enhancement of said molecules to enhance said signal on single molecule level, allowing single molecule analysis at concentrations of the compounds to be measured at picomolar or nanomolar or smaller concentration. A first object is to provide a method for amplifying a signal based on a single target molecule. In addition, kits or systems are provided allowing single molecule analysis based on single enhancement.

### Brief description of the present invention

In a first aspect, a method for amplifying a signal based on a single target molecule containing a target sequence comprising the steps of:
a) providing an array comprising a multiple of nucleic acid molecules;
b) providing a first molecule having a single strand nucleic acid sequence binding specifically to a target sequence whereby said single stranded nucleic acid sequence has a first domain comprising a nicking enzyme cleavage site and a second domain binding specifically to a first nucleic acid sequence present on the array, and having a quencher moiety located on the opposite side of the first domain in relation to the second domain binding said first molecule specifically to the first nucleic acid sequence present on the array, and optionally, the first molecule has a label, said label is quenched when the quencher moiety is present at this molecule;
c) binding specifically of the first molecule to the nucleic acid molecule having the first sequence present on the array;
d) providing of a second molecule having a single strand nucleic acid sequence with a first domain hybridizing specifically to the first domain comprising the nicking enzyme cleavage site of the first molecule, thus, providing an active cleavage site of the nicking enzyme after binding and forming a double stranded nucleic acid sequence with the first domain of the first molecule, and optionally, with a second domain containing the target sequence, whereby this step d) may be conducted before providing the first molecule according to step b);
e) binding of the second molecule to the first molecule, thus, forming an active cleavage site of the nicking enzyme;
f) optionally, providing third molecules having a single stranded nucleic acid sequence with a first domain comprising a nicking enzyme cleavage site, a second domain binding to nucleic acid sequences present on the array being different to the first sequence present on the array, and having a quencher moiety located on the opposite side of said first domain in relation to the second domain, and optionally the third molecule has a label, said label is quenched when the quencher moiety is present at this molecule;
g) cleavage of the single stranded nucleic acid strand of the first molecule at the cleavage site of the nicking enzyme, thus, cleaving off the quencher moiety from the first molecule;
h) binding of further first or third molecules located adjacent to the second molecule with said second molecule partially bound to the remaining part of the first molecule;
i) migration of the second molecule from the first molecule to a further first or third molecule having a quencher moiety;
j) optionally repeating steps g) to i), thus, cleaving the quencher moieties from molecules bound to nucleic acid sequences adjacent to the first sequence present on the array;
   optionally, when the label is not present on the first or third molecule, further comprising providing an imager molecule having a single stranded nucleic acid sequence for binding of the imager molecule to the first or third molecule,
   whereby the imager molecule has a label at one end, said label is quenched when a quencher moiety is present at the first or third molecule; and,
k) measuring the signal based on single target molecules by measuring the label,
whereby the single target molecule containing a target sequence may be present on the array or may be provided in form of a second molecule, is provided.

In a further aspect, the present invention relates to a kit or system for signal enhancement of a label, like chromophores, in particular fluorophores. Finally, the use of a method according to the present invention or a kit or system according to the present invention for signal enhancement, in particular, enhancement of fluorescence and/or for single molecular analysis and/or for use in living cells, optionally, said living cells are genetically cells expressing a nicking enzyme, are provided.

### Brief description of the drawings

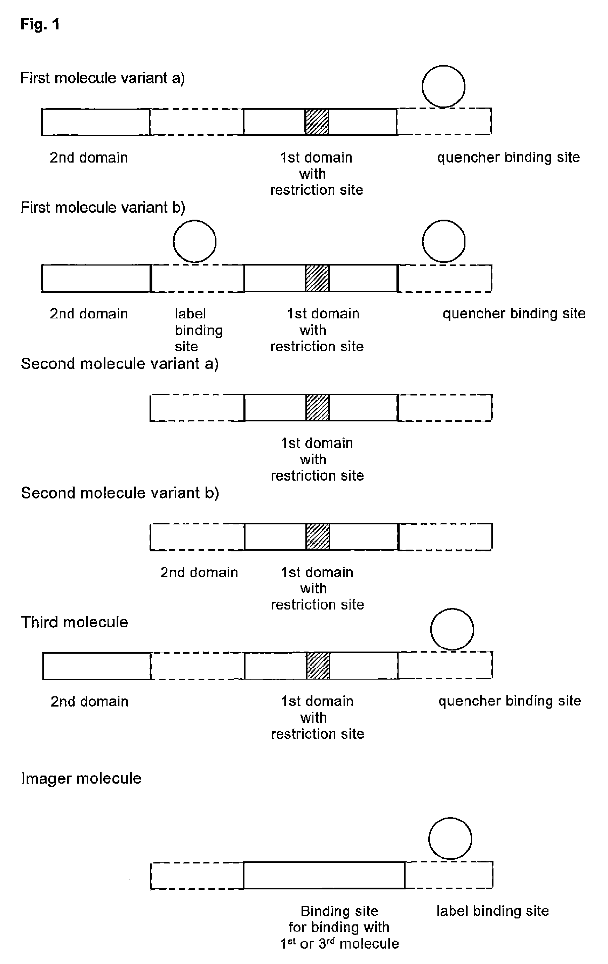

In figure 1 the different types of molecules are shown. That is, for the first molecule two variants are described. In variant a) the quencher moiety is present with the first molecule further comprising the first domain with a restriction site and the second domain for binding specifically to a first nucleic acid sequence present on the array. As shown, the length of the various domains may vary including the domains located between the first and second domain as well as the other domain where the quencher moiety is bound to opposite to the second domain in relation to the first domain.

Further, a first molecule of variant b) is shown. Therein, a further site is present where the label is bound to. This variant reflects the possible hairpin structure of the first molecule. When the hairpin structure is formed, complementary sequences in the domain of the quencher binding site and the domain of the labelling binding site or located somewhere in between the two moieties of label and quencher binding site, are hybridized, thus forming the hairpin structure, while the second domain is at the free end of the hairpin structure allowing hybridization with the nucleic acid sequence present on the array accordingly.

In addition, a second molecule of variant a) is shown comprising the first domain with the restriction site for the nicking enzyme having further moieties at the left and right side thereof.

The second molecule variant b) comprises additionally a second domain allowing binding of the second molecule to contain a target sequence for binding thereof at a target binding sequence present on the array.

Further, the structure of the third molecule is shown comprising the second domain allowing binding to a nucleic acid sequence present on the array, a first domain with the restriction site for the nicking enzyme as well as a further structure where the quencher moiety is bound to. With the dotted lines it is indicated that the length of the domains may vary.

Finally, the imager molecule is shown containing the binding site for binding with the first or third molecule as well as the further domain containing the label accordingly.

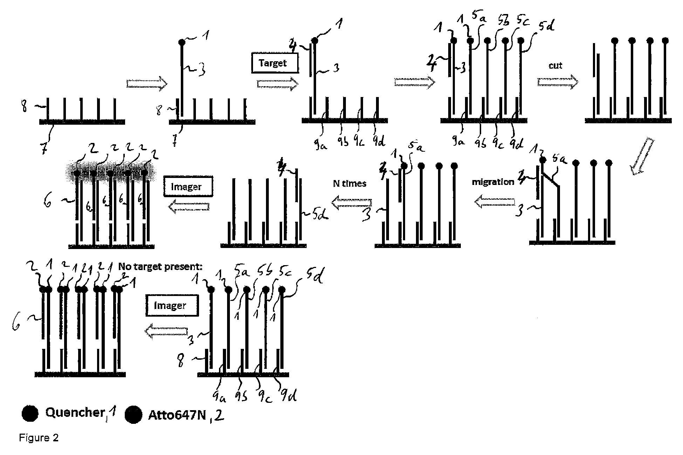

Figure 2 shows a sequence of autonomous DNA walking for linear signal amplification.

Therein, the array 7 is shown comprising the first nucleic acid sequence 8. The first sequence 3 comprising the quencher 1 binds to the first nucleic acid sequence 8 on the array 7. After binding, the second sequence here also called the target 4, hybridizes to the first sequence 3, thus, forming the restriction site for the nicking enzyme. After binding of the target molecule 4 to the first molecule 3, third molecules 5a to 5d including quencher moieties 1 bind to further nucleic acid sequences 9a, 9b, 9c, 9d present on the array. Thereafter, the nicking enzyme is added, thus, allowing cutting of the first molecule 3 and releasing the quencher moiety 1 accordingly. After cutting, hybridisation of the third molecule 5a with the free sequence of the second sequence 4 occurs by toehold binding. The second sequence 4 migrate to the third sequence 5a, thus, forming a further restriction site for the nicking enzyme and the quencher molecule 1 present on the third molecule 5a is cut off. This is repeated several times, thus, releasing all quencher molecules1 present on the first and third molecules accordingly.

Thereafter, the imager molecule 6 is added hybridizing to the first and third molecules accordingly. The imager molecule 6 contains the label 2, thus, the initial target with the single target sequence allows for signal amplification, in the present figure 2 a signal amplification of five times.

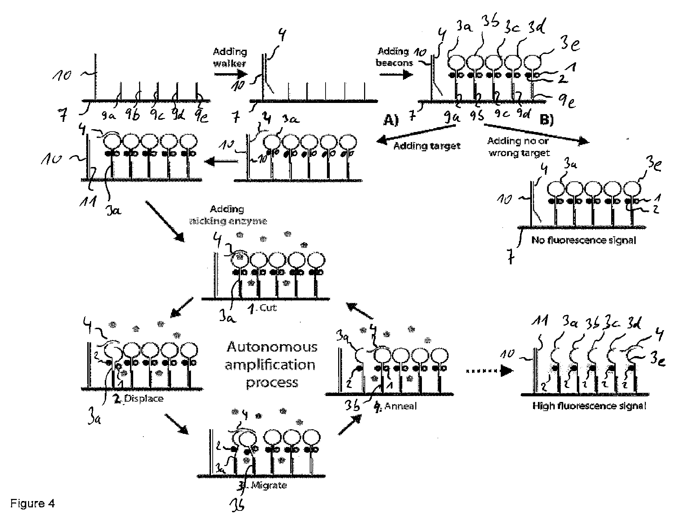

In the lower row, the situation where no target and, thus, no cutting occurs, is shown. The imager molecule 6 hybridized to the first 3 and third molecule 5a, ..containing the label moiety. However, due to the intimidate presence of the quencher molecule 1, the label 2 is quenched and thus, no signal can be determined. Of note, as with figure 4, the reference signs in the sequence of the plots are not identified and, thus, are not shown with each of the plots.

Figure 3 shows the results of a DNA walker experiment on an array system showing the intensity. As shown in figure 3a, the intensity is shown with the DNA walker system while in figures 3b and 3c the same system without the target, without the second sequence as well as without the enzyme is shown. It is clear from the histograms, that the signal intensity can be amplified and enhanced accordingly.

In figure 4 a further embodiment of the present invention is shown. Here, the first sequence 3 is present in variant b) according to figure 1 able to form a hairpin structure.

An array 7 is provided comprising a target binding sequence 10 where the second sequence 4 (walker) hybridizes to. The array comprises further nucleic acid sequences 9a, 9b, ... allowing binding of the first sequence 3a, 3b, ... forming a hairpin structure wherein the quencher moiety 1 is adjacent to the label moiety 2, thus, quenching the label 2 accordingly. The domain containing the restriction site for the nicking enzyme is present in the hairpin structure. Binding of the first molecule 3 to the array is via the second domain of the first molecule binding to the further nucleic acid sequence 9 as present on the array 7.

Adding the target molecule in form of an invader sequence 11 results in displacement of the second sequence 4 bound to the target binding sequence 10 with the invader sequence 11 containing the target. As shown, the sequence of the second molecule 4 binding to the hairpin structure of the first molecule 3 is located in the lower part, i.e. the array structure and not at the free ends of the target binding sequence 10. Thus, it is possible to have a sufficient distance between the complementary sequences present on the second molecule, the walker, and the hairpin structure of the first molecule. Only in the presence of the invader sequence 11, the second sequence 4 migrates to the first sequence 3a by hybridizing to the hairpin structure forming the full and functional restriction site for the nicking enzyme while in case of not adding the invader sequence 11, variant b), no migration of the second sequence 4 occurs and, thus, no fluorescence signal due to quenching of the fluorescence label occurs.

In the presence of the invader sequence representing the target in the present case the migration of the sequence occurs. When adding the nicking enzyme, cutting of the first sequence in form of the hairpin structure occurs due to formation of the full restriction site by the first and second molecule. An autonomous amplification step with displacement, migration, annealing and cutting happens, thus, the single presence of the target binding sequence 10 is amplified by obtaining a higher fluorescence signal accordingly.

### Detailed description of the present invention

In a first aspect, the present invention relates to a method for amplifying a signal based on a single target molecule containing a target sequence comprising the steps of:
a) providing an array comprising a multiple of nucleic acid molecules;
b) providing a first molecule having a single strand nucleic acid sequence binding specifically to a target sequence whereby said single stranded nucleic acid sequence has a first domain comprising a nicking enzyme cleavage site and a second domain binding specifically to a first nucleic acid sequence present on the array, and having a quencher moiety located on the opposite side of the first domain in relation to the second domain binding said first molecule specifically to the first nucleic acid sequence present on the array, and optionally, the first molecule has a label, said label is quenched when the quencher moiety is present at this molecule;
c) binding specifically of the first molecule to the nucleic acid molecule having the first sequence present on the array;
d) providing of a second molecule having a single strand nucleic acid sequence with a first domain hybridizing specifically to the first domain comprising the nicking enzyme cleavage site of the first molecule, thus, providing an active cleavage site of the nicking enzyme after binding and forming a double stranded nucleic acid sequence with the first domain of the first molecule, and optionally, with a second domain containing the target sequence, whereby this step d) may be conducted before providing the first molecule according to step b);
e) binding of the second molecule to the first molecule, thus, forming an active cleavage site of the nicking enzyme;
f) optionally, providing third molecules having a single stranded nucleic acid sequence with a first domain comprising a nicking enzyme cleavage site, a second domain binding to nucleic acid sequences present on the array being different to the first sequence present on the array, and having a quencher moiety located on the opposite side of said first domain in relation to the second domain, and optionally the third molecule has a label, said label is quenched when the quencher moiety is present at this molecule;
g) cleavage of the single stranded nucleic acid strand of the first molecule at the cleavage site of the nicking enzyme, thus, cleaving off the quencher moiety from the first molecule;
h) binding of further first or third molecules located adjacent to the second molecule with said second molecule partially bound to the remaining part of the first molecule;
i) migration of the second molecule from the first molecule to a further first or third molecule having a quencher moiety;
j) optionally repeating steps g) to i), thus, cleaving the quencher moieties from molecules bound to nucleic acid sequences adjacent to the first sequence present on the array;
   optionally, further comprising providing an imager molecule having a single stranded nucleic acid sequence for binding of the imager molecule to the first or third molecule, whereby the imager molecule has a label at one end, said label is quenched when a quencher moiety is present at the first or third molecule when the label is not present on the first or third molecule; and,
k) measuring the signal based on single target molecules by measuring the label,
whereby the single target molecule containing a target sequence may be present on the array or may be provided in form of a second molecule.

That is, the present inventors successfully arrived at a method for enhancing the signal of a single target molecule by way of the DNA walker technique. The present inventors aims in providing methods for enhancing and amplifying a signal based on a single target molecule whereby the signal is enhanced locally, thus, allowing specific detection even at low concentrations. It was surprising to provide a method wherein a molecule containing a nucleic acid strand is able to move from one molecule to a further molecule, thus, allowing to increase the label signal, like the fluorescence signal, of the single molecule. For example, if the target sequence is present with the second molecule, this molecule representing the walker can enhance or amplify the signal by "activating" various label, e.g. by releasing the quencher adjacent to a label.

In this connection, the terms "enhancement" or "enhancing" and "amplification" or "amplifying" are used interchangeably unless otherwise defined. On the other hand, if in case of an array, like an nucleic acid based origami containing different types of nucleic acid molecules, the targeting sequence is a specific nucleic acid sequence while adjacent nucleic acid sequences are different but all nucleic acid molecules present on the array may have an identical domain. The third molecule as mentioned above may bind to this common domain of the nucleic acid sequences present on the array, thus, allowing to amplify the fluorescence signal by the DNA walker technique according to the present invention.

The term "array" as used herein, refers to a substrate containing e.g. multiple of nucleic acid molecules. In one embodiment of the present invention, the array is a nucleic acid based origami molecule or a nucleic acid based chip. For example, the array is a DNA origami containing various single stranded DNA extensions being arranged adjacent to one another. It is preferred, that the distance between these multiple nucleic acid molecules present on the array, for example in form of single stranded DNA extensions present in a DNA origami is in the field of 1 nm to 12 nm, like 2 nm to 10 nm, for example 4 nm to 8 nm, like 5 nm to 6 nm.

The method is based on the amplification of a signal, namely an amplification, by removing a quencher moiety next to a label, thus, allowing the amplification or enhancement of the signal by amplifying the number of label to be measured accordingly.

That is, the method is based on a cleavage of the molecule containing the quencher moiety by using a nicking enzyme whereby the restriction site for cleavage is formed only when the walker, the second molecule, forms the double stranded DNA required.

A nicking enzyme, also known as a nicking endonuclease, is an enzyme that cuts one strand of a double stranded DNA at a specific recognition nucleotide sequence known as a restriction site. Such enzymes hydrolyze or cut only one strand of the DNA duplex to produce DNA molecules that are nicked rather than cleaved. In case of the present invention, the nicking enzyme is used to cut the first or third molecule in a way that the quencher is released, thus, enable detection of a label present with the first or third molecule or present with an imager molecule bound to the first or third molecule accordingly.

The term "binding" or "bound" as used herein includes the embodiment of hybridization. That is, binding of nucleic acid sequences to one another, also known as hybridization, are based on specific interaction of complementary bases present in the nucleic acid sequence. Hybridization takes place at appropriate conditions allowing specific hybridization accordingly. The skilled person is well aware of the suitable hybridization conditions.

The label which signal is amplified is typically a chromophore like a fluorophore. The skilled person is well aware of suitable chromophores including fluorophores.

Typically, fluorescent dyes are used in fluorescence based techniques including the label/quencher system of ATTO647N and BBQ650.

The term "nucleic acid molecules" includes also structures containing parts or consisting of other nucleic acid structures, like RNA, PNA etc.,. The skilled person is well aware of suitable nucleic acid molecules which can be employed in the present invention.

The method according to the present invention comprises the use of an array, which may be nucleic acid based structures of predefined shape like nucleic acid based origami or single stranded tiles (SST) structures, a first molecule, a second molecule, optionally a third molecule as well as optionally imager molecules. In addition, a nicking enzyme is required as well as means for measuring the signal.

The first molecule is a molecule having a single stranded nucleic acid sequence. In addition, the first molecule has a quencher moiety at one side of the molecule. For example, the first molecule is a molecule having the following moieties, see figure 1:

A first domain comprising a nicking enzyme cleavage site, a second domain binding specifically to a nucleic acid sequence present on the array, a quencher moiety being located on the opposite site of the second domain in relation to the first domain. The quencher molecule may be present at the far end or distal end of the molecule but may also be present at the peripheral end of said molecule. In addition, the first molecule may in some embodiments contain additionally a label whereby said label is quenched when the quencher moiety is present at the first molecule. In one embodiment, the first molecule contains a hairpin structure whereby the quencher and label are located next to each other, thus, allowing quenching of the label by the quencher accordingly.

The second molecule is a molecule having a single strand nucleic acid sequence. The second molecule comprises a first domain hybridizing specifically to a first domain comprising the nicking enzyme cleavage site of the first molecule, thus, upon hybridization, an active cleavage site of the nicking enzyme is formed by binding and forming a double stranded nucleic acid sequence composed of the first domain of the first molecule and a first domain from the second molecule.

In an embodiment, the second molecule comprises a further domain, called a second domain, containing the target sequence. For example, the target sequence may be part of the target or may be a sequence determining the target specifically, e. g. hybridizing specifically to the target, see figure 1.

The second molecule may be provided either before providing the first molecule or may be provided after providing and binding of the first molecule to the array. In one aspect, the second molecule representing the DNA walker molecule may bind to a nucleic acid sequence present on the array on a single strand nucleic acid sequence present thereon accordingly. The first molecule and, optionally, the third molecule are then added to the system thus allowing binding thereof to other single stranded nucleic acid sequences present on the array like the nucleic acid based origami. In one aspect, the second molecule is then subject to a strand displacement. This means, that a further nucleic acid sequence, e.g. an invader sequence, is provided displacing or replacing the bound second molecule from the complementary strand due to higher binding strength, e. g. having more specific complementary bases. The displaced second molecule can then "walk" to the adjacent first or third molecule, thus, forming a double stranded sequence. This double-stranded sequence comprises the nick enzyme cleavage site allowing cleavage of the first or third molecule and, consequently, releasing the domain containing the quencher moiety from the first or third molecule. As a consequence, the label is no longer quenched and can be determined accordingly. In an alternative embodiment of the method according to the present invention, the second molecule is provided after binding of the first or third molecules without interaction of the second molecule with the nucleic acid sequences present on the array. The second molecule interact, e.g. hybridize, to the first and/or third molecule specifically, thus, forming the active nicking enzyme cleavage site. Thereafter, the nicking enzyme is added and the walking machinery can start.

The walking or displacement may be based on the so called toehold mediated displacement. In this reaction, an invader DNA strand reacts with the single-stranded part (toehold) of a partly double-stranded DNA and then displaces one strand by strand displacement.

An incumbent strand is initially hybridized to a complementary 'displacement' domain of a target strand by Watson-Crick base pairing. The invader strand is complementary both to the displacement domain of the target and to an adjacent 'toehold' domain, allowing it to displace the incumbent to form a more stable duplex. Displacement can be initiated by binding of the invader to the toehold followed by a branch migration process in which base pairs between target and incumbent break and are replaced by base pairs with the invader.

In the embodiment wherein the first and/or third molecule form a hairpin structure or other structures where quenching of the label is given in this molecule itself, the addition of an additional imager molecule may not be necessary but, after cleavage with the nicking enzyme, the quenching moiety is released. In case of an embodiment wherein the first or third molecule do not comprise both, the quencher and the label moiety, the imager molecule may be added enabling binding of the same to the first and/or third molecule. Only in case where the quenching moiety with the quencher is cleaved off, the signal of the imager molecule can be determined.

The third molecule is a molecule having a single stranded nucleic acid sequence with a first domain comprising a nicking enzyme cleavage site, a second domain binding to nucleic acid sequences present on the array being different to the first sequence present on the array, and having a quencher moiety located on the opposite site of said first domain in relation to the second domain. Optionally, the third molecule has a label, said label is quenched when the quencher moiety is present at this molecule.

In an aspect, the third molecule may comprise a domain with a nucleic acid sequence complementary in hybridizing specifically to a common nucleic acid sequence present on each of the nucleic acid molecules of the array. That is, the third molecule is a molecule which can occupy each single nucleic acid sequences present on the array or extend in particular, extending from the nucleic acid based origami accordingly.

A DNA origami is a DNA nanostructure formed from one long scaffold strand and many small, so-called staple strands. Alternatively, the array can be composed of alternative DNA nanostructures such as single-stranded tiles or gridiron structures or related, see e.g. Bryan Wei, Mingjie Dai & Peng Yin doi:10.1038/nature11075 nature 2012. Of course other nucleic acid based structures are possible as well as substitutes of DNA, like RNA, LNA.

The method according to the present invention comprises cleavage of the single strand nucleic acid strand of the first and/or third molecule at the cleavage site of the nicking enzyme. The nicking enzyme may be added after formation of the cleavage site or may be already present in the system. Typically, the nicking enzyme is added after formation of the double stranded restriction site formed by the first and second molecule accordingly.

The nicking enzyme cleaves the quencher moiety from the first molecule and/or third molecule, thus, allow a detection of the label being no longer quenched.

After release of the quencher moiety, the second molecule can migrate from the first molecule to a further first or third molecule adjacent to the present molecule. This DNA walking is possible by toehold mediated strands displacement and is also known as DNA walking. The method optionally include repeating the steps of binding of the second molecule for forming the restriction site, cleavage by the nicking enzyme resulting in the release of the quencher molecule and further migration to the adjacent sequences, thus, allowing enhancement and amplification of the signals locally.

In addition, in an embodiment of the present invention it is possible to use this walking mechanism to activate the label in an enhanced electromagnetic field e.g. of plasmon resonance formed e.g. by nanoantennas, e.g. described in Puchkova, A. et. al., see above.

That is, using this walking mechanism, it is possible to bring the label into an electrical field enhancement formed e.g. by plasmon resonance, thus, amplifying the signal based on a single target molecule further.

The method according to the present invention allows for multiplex amplification. That is, the nucleic acid based nanostructure, for example in form of a DNA origami, may be equipped with a label creating a bar code based on different numbers of labels or of different numbers, thus, having e.g. different colors etc. For example, the different targets are detected with different colors, thus, allowing to create a kind of bar code or for determining the presence of different targets at the same time.

In an embodiment of the present invention, in particular, when using imager molecules, the nicking enzyme is removed or inactivated totally before providing said imager molecules.

Typically, the length of the first, second and/or third molecule is at least 20 nucleic acids, like at least 25 nucleic acids and at most 60 nucleic acids, like at most 50 nucleic acids.

The skilled person is well aware of suitable sizes of the different molecules enabling specific hybridization accordingly.

In an embodiment of the present invention, the array is a nucleic acid based origami with metallic nanoparticles being bound thereto whereby the nanoparticles are arranged to allow formation of electrical field enhancement by plasmon resonance and the label for measuring the signal are positions in the area of electrical field enhancement due to plasmon resonance accordingly.

Further, the present inventions provides in an embodiment a method wherein the array comprising a multiple of nucleic acid molecules is an array having different nucleic acid molecules and the first molecule and the third molecule hybridize two different nucleic acid sequences present on said array. In addition, the second molecule may be composed of different types of second molecules enabling specific hybridization with different types of first and/or third molecules. Thus, multiplex amplification is possible, e.g. for bar coding etc.

In an embodiment, the first and/or third molecules are molecules having a hairpin structure, whereby the label and the quencher are present at the neck of the hairpin structure, thus, the label is quenched, and the first domain containing the nicking enzyme cleavage site is located in the loop of the hairpin in between the binding site of the label and the quencher. In an embodiment, the method according the present invention is an isothermal method, that is, it is not required to heat and cool the system in cycles, like it is required when performing polymerase chain reaction. That is, during the various steps of the method according to the present invention, the system is maintained at a substantially unchanged temperature.

In addition, the method according of the present invention can be used to determine mismatches including single nucleotide polymorphisms. That is, under appropriate conditions, namely under appropriate hybridization conditions, the second molecule with the specific target sequence allows to differentiate between fully complementary sequences or sequences having one or two or three etc. mismatches. That is, depending on the number of mismatches, the walking step may occur with a less probability in case of higher mismatches. The signal of enhancement depends on the number of mismatches between the target sequences and the complementary sequence present on the complementary strand of the other molecule, like the first or third molecule accordingly.

The method according to the present invention is useful for various purposes and approaches including determining analytes at low concentrations of picomolar or nanomolar ranges, e.g. single molecule level in a sample, for diagnostics based on biomarker as well as in the field of chip technology. In addition, the method can be used to determine polymorphisms and for signal amplification in general.

In a further aspect, the present invention relates to a kit or system for signal enhancement of label or marker including chromophores, in particular fluorophores comprising first molecules having a single strand nucleic acid sequence binding specifically to a sequence whereby said single stranded nucleic acid sequence has a first domain comprising a nicking enzyme cleavage site and a second domain binding specifically to a first nucleic acid sequence, and having a quencher moiety located on the opposite side of the first domain in relation to the second domain said first domain binding specifically to a first nucleic acid sequence, and optionally, the first molecule has a label, said label is quenched when the quencher moiety is present at this molecule; second molecules having a single strand nucleic acid sequence with a domain hybridizing specifically, thus, binding, to the first domain comprising the nicking enzyme cleavage site of the first molecule for providing an active cleavage site of the nicking enzyme after binding and forming a double stranded nucleic acid sequence with the first domain of the first molecule and, optionally, with a second domain containing the target sequence; optionally, third molecules having a single stranded nucleic acid sequence with a first domain comprising a nicking enzyme cleavage site, a second domain binding to a nucleic acid sequence being different to the first nucleic acid sequence present; and having a quencher moiety located on the opposite side of said first domain in relation to the second domain, and, optionally, the first molecule has a label, said label is quenched when the quencher moiety is present at this molecule; optionally, imager molecules having a single stranded nucleic acid sequence for binding of this molecule to the first or third molecule whereby the imager molecule has a chromophore, in particular, a fluorophore, at one end, said chromophore, in particular, a fluorophore, is quenched when a quencher moiety is present at the first or third molecule; and optionally, a nicking enzyme.

The system or kit according to the present invention is useful for signal enhancement, in particular, enhancement of fluorescence, in molecular diagnostics, and/or in single molecule analysis and/or in living cells, optionally, said living cells are genetically engineered cells expressing the nicking enzyme.

In an embodiment of the present invention, said kit or system comprises further the array, e.g. in form of a nucleic acid based origami. The nucleic acid based origami may further comprise means for allowing electrical field enhancement of the signal e.g. by plasmon resonance.

That is, in an embodiment of the present invention, the first, second and third molecule, present in the kit or system, have a size of 20 to 60 nucleic acid length. In an embodiment, the first domains present in the first molecule and third molecule are identical.

In addition, in an embodiment of the present invention, the first molecule and the third molecule have a common sequence for allowing binding to a complementary common sequence present in the multiple of nucleic acid molecules on the array. In addition, in an embodiment of the present invention the kit or system further comprises a nucleic acid based origami as the array comprising a multiple of nucleic acid molecules optionally having different nucleic acid sequences whereby these multiple nucleic acid molecules are single stranded nucleic acid molecules extending from the nucleic acid based origami accordingly. This origami may further comprise metallic nanoparticles being arranged on the array to allow formation of an array of electrical field enhancement due to plasmon resonance.

The method and system according to the present invention allows to enhance or amplify a signal, e.g. a signal based on a chromophore like a fluorophore. Of course, the signal may be derived from other means including radioactivity, Raman spectroscopy, labels for AFM imaging, etc.

Due to this local and specific enhancement or amplification of the signal, the signal representing a single target molecule can be determined even at low levels.

The present invention is based on a local amplification of the signal by a cascade based on the DNA walker technique. In an embodiment, this kind of amplification or enhancement can be combined with other known techniques, including plasmon resonance etc. The system and method can be conducted easily without expensive equipment and laborious working steps. In addition, the method and system do not require cycling conditions as it is necessary in the PCR method.

That is, the method and system according to the present invention allows a fast and easy detection of target molecules at low concentrations as required in molecular diagnostics.

The present invention will be described by example further without restricting the same thereto.

### Examples

### Example 1

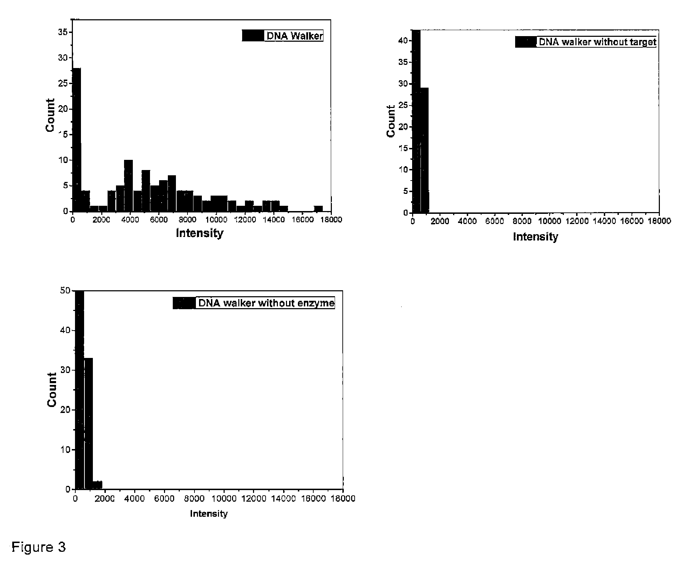

A rectangular DNA origami containing an Atto542 label for recognition and 166 binding sites was functionalized as an array and bound to a neutravidin BSA-biotin surface. The binding occurs via further biotinylated staple strands incorporated in the DNA origami. 10 nM starting DNA (first molecule variant a) was added for incubation for 1 hour at room temperature. Excess starting staples were removed by washing six times with PBS PBS (137 mM sodium chloride, 2.7 mM potassium chloride, 10 mM disodium phosphate and 1.8 mM monopotassium phosphate) containing 12.5 mM MgCl₂ and 0.01% Tween 20. 10 nM target DNA (second molecule variant a) was incubated to bind to the starting staple for 1 hour at room temperature. Excess target DNA was removed by washing six times with the PBS mentioned above. 30 nM walker binding DNA (third molecule) was incubated for 1.5 hours at room temperature. Excess walker binding DNA was removed by washing six times with the PBS mentioned above. 100 *µ*l PBS containing 12.5 mM MgCl₂, 10 *µ*l 1X CutSmart® Buffer and 1 *µ*l DNA nicking enzyme Nb.Btsl was incubated for 2 hours at room temperature. PBS containing 330 mM NaCl and 0.02% Paraformaldehyde was incubated for 3 minutes to inactivate the nicking enzyme. Excess paraformaldehyde was removed by washing with PBS containing 330 mM NaCl and 0.01% Tween 20. 5 nM imager molecule in PBS containing 330 mM NaCl and 0.01% Tween 20 was incubated for 1 hour and excess was removed by washing three times with PBS containing 330 mM NaCl and 0.01% Tween 20. Atto647N was employed as fluorophore in the imager molecule while BBQ650 was employed as quencher in the first and third molecule. Single molecule confocal measurements were performed with a 80 MHz pulsed red laser at 640 nm and 1 µW excitation and a green laser at 532 nm and 2 µW excitation power. The single molecule surface was scanned with a Piezo-Stage and the fluorescence signal was detected by Single-Photon Avalanche Diodes and registered by a TCSPC PC card. For intensity analysis, co-localized (red and green) single molecule spots were selected, the red intensity was extracted and plotted as histogram (see figure 3).

Control measurements were performed without incubation of target DNA (second molecule) and without incubation of nicking enzyme.

Single-nucleotide polymorphism experiments were performed as described above with the difference that the rectangular DNA origami contained only 5 instead of 166 binding sites. The experiments were conducted with different target DNA (second molecule incorporating no, 1, 2 or 3 mismatched nucleotides in the domain after the restriction site). It was possible to demonstrate that with one or more mismatches, the signal is lowerd. Further, it was possible to demonstrate that depending on the number of mismatches the signal intensity decreased. Thus, it is possible to demonstrate that the method and system according to the present invention represents a suitable measure for single nucleotide polymorphism experiments.

## Claims

1. A method for amplifying a signal based on a single target molecule containing a target sequence comprising the steps of:
a) providing an array comprising a multiple of nucleic acid molecules;
b) providing a first molecule having a single strand nucleic acid sequence binding specifically to a target sequence whereby said single stranded nucleic acid sequence has a first domain comprising a nicking enzyme cleavage site and a second domain binding specifically to a first nucleic acid sequence present on the array, and having a quencher moiety located on the opposite side of the first domain in relation to the second domain binding said first molecule specifically to the first nucleic acid sequence present on the array, and optionally, the first molecule has a label, said label is quenched when the quencher moiety is present at this molecule;
c) binding specifically of the first molecule to the nucleic acid molecule having the first sequence present on the array;
d) providing of a second molecule having a single strand nucleic acid sequence with a first domain hybridizing specifically to the first domain comprising the nicking enzyme cleavage site of the first molecule, thus, providing an active cleavage site of the nicking enzyme after binding and forming a double stranded nucleic acid sequence with the first domain of the first molecule, and optionally, with a second domain containing the target sequence, whereby this step d) may be conducted before providing the first molecule according to step b);
e) binding of the second molecule to the first molecule, thus, forming an active cleavage site of the nicking enzyme;
f) optionally, providing third molecules having a single stranded nucleic acid sequence with a first domain comprising a nicking enzyme cleavage site, a second domain binding to nucleic acid sequences present on the array being different to the first sequence present on the array, and having a quencher moiety located on the opposite side of said first domain in relation to the second domain, and optionally the third molecule has a label, said label is quenched when the quencher moiety is present at this molecule;
g) cleavage of the single stranded nucleic acid strand of the first molecule at the cleavage site of the nicking enzyme, thus, cleaving off the quencher moiety from the first molecule;
h) binding of further first or third molecules located adjacent to the second molecule with said second molecule partially bound to the remaining part of the first molecule;
i) migration of the second molecule from the first molecule to a further first or third molecule having a quencher moiety;
j) optionally repeating steps g) to i), thus, cleaving the quencher moieties from molecules bound to nucleic acid sequences adjacent to the first sequence present on the array;
optionally, further comprising providing an imager molecule having a single stranded nucleic acid sequence for binding of the imager molecule to the first or third molecule, whereby the imager molecule has a label at one end, said label is quenched when a quencher moiety is present at the first or third molecule, when the label is not present on the first or third molecule; and,
k) measuring the signal based on single target molecules by measuring the label,
whereby the single target molecule containing a target sequence may be present on the array or may be provided in form of a second molecule.

2. The method according to claim 1 wherein the label is a chromophore, in particular, a fluorophore.

3. The method according to claim 1 or 2 wherein the length of the second molecule is at least 20 nucleic acids, like at least 25 nucleic acids and at most 60 nucleic acids like at most 50 nucleic acids.

4. The method according to any one of the preceding claims wherein the nicking enzyme is removed totally before providing the imager molecule.

5. The method according to any one of the preceding claims for multiplex amplification.

6. The method according to any one of the preceding claims further comprising the step of amplifying the signal based on a single target molecule by electrical field enhancement, like plasmon resonance.

7. The method according to any one of the preceding claims wherein the array comprising a multiple of nucleic acid molecules is a nucleic acid based origami structure.

8. The method according to any one of claims 6 or 7 comprising nucleic acid based origami with metallic nanoparticles being bound thereto whereby the nanoparticles are arranged to allow formation of plasmon resonance and wherein the label for measuring the signal are positioned in the area of electrical field enhancement due to plasmon resonance.

9. The method according to any one of the preceding claims wherein the array comprising a multiple of nucleic acid molecules is an array having different nucleic acid molecules and the first molecule and the third molecule contain different nucleic acid sequences present on said array.

10. The method according to any one of the preceding claims wherein the first and/or third molecules are molecules having a hairpin structure, whereby the label and the quencher are present at the neck of the hairpin structure, thus, the label is quenched and the first domain containing the nicking enzyme cleavage site is located in the loop of the hairpin.

11. A kit or system for signal enhancement of chromophores, in particular fluorophores, comprising
first molecules having a single strand nucleic acid sequence binding specifically to a sequence whereby said single stranded nucleic acid sequence has a first domain comprising a nicking enzyme cleavage site and a second domain binding specifically to a first nucleic acid sequence, and having a quencher moiety located on the opposite side of the first domain in relation to the second domain said first domainbinding specifically to a first nucleic acid sequence, and optionally, the first molecule has a label, said label is quenched when the quencher moiety is present at this molecule;
second molecules having a single strand nucleic acid sequence with a domain hybridizing specifically, thus, binding, to the first domain comprising the nicking enzyme cleavage site of the first molecule for providing an active cleavage site of the nicking enzyme after binding and forming a double stranded nucleic acid sequence with the first domain of the first molecule and, optionally, with a second domain containing the target sequence;
optionally, third molecules having a single stranded nucleic acid sequence with a first domain comprising a nicking enzyme cleavage site, in a second domain binding to a nucleic acid sequence being different to the first nucleic acid sequence present; and having a quencher moiety located on the opposite side of said first domain in relation to the second domain, and, optionally, the first molecule has a label, said label is quenched when the quencher moiety is present at this molecule;
optionally, imager molecules having a single stranded nucleic acid sequence for binding of this molecule to the first or third molecule whereby the imager molecule has a chromophore, in particular, a fluorophore, at one end, said chromophore, in particular, a fluorophore, is quenched when a quencher moiety is present at the first or third molecule;
and optionally, a nicking enzyme.

12. The kit or system according to claim 11 wherein the first, second and third molecules have a size of 20 to 60 nucleic acid length.

13. The kit or system according to claim 11 or 12 wherein the first domains present in the first molecule, second molecule and third molecule are identical.

14. The kit or system according to any one of claims 11 to 13 wherein the first molecule and the third molecule have a common sequence for allowing binding to a complementary common sequence present in the multiple of nucleic acid molecules on the array.

15. The kit or system according to any one of claims 11 to 14 wherein the kit further comprises a nucleic acid based origami as the array comprising a multiple of nucleic acid molecules optionally, having different nucleic acid sequences, and/or comprising metallic nanoparticles being arranged on the array to allow formation of an area of electrical field enhancement due to plasmon resonance.

16. The use of a method according to any one of claims 1 to 10, a kit or system according to claim 11 to 15 for i) signal enhancement, in particular, enhancement of fluorescence, and/or ii) in single molecule analysis and/or iii) in living cells, optionally, said living cells are genetically engineered cells expressing the nicking enzyme.
